# EUROPEAN PATENT APPLICATION

(11) **EP 4 772 621 A1**
(43) Date of publication of application: **08.07.2026**
(21) Application number: 24835480.5
(22) Date of filing: 21.08.2024
(51) Int. Cl.: C12N 9/22, C12N 15/55

(54) **BENZONASE NUCLEASE VARIANT WITH IMPROVED SALT TOLERANCE AND USE THEREOF**

(30) Priority: 26.09.2023 CN 202311249502
(71) Applicant: Beijing Acrobiosystems Biotechnology Co., Ltd, Beijing 100176 (CN)
(72) Inventor: TIAN, Li, Beijing 100176 (CN); WANG, Lei, Beijing 100176 (CN); QIN, Lili, Beijing 100176 (CN); ZHANG, Miaomiao, Beijing 100176 (CN); GONG, Bingxue, Beijing 100176 (CN); JI, Shuxian, Beijing 100176 (CN); CHEN, Yiding, Beijing 100176 (CN); MIAO, Jingyun, Beijing 100176 (CN)
(74) Representative: Vogelbruch, Keang
(86) International application number: PCT/CN2024/113570
(87) International publication number: WO 2025/008002

(57) **Abstract**

The present application relates to the technical field of enzyme engineering. Specifically provided is a benzonase nuclease variant. Compared with a wild-type benzonase nuclease, the variant can tolerate a wider salt concentration range, and maintains a good enzyme activity under an elevated salt concentration, which expands the use of benzonase nuclease under different salt concentrations.

## Description

### CROSS-REFERENCE TO THE RELATED APPLICATIONS

The present invention claims priority to Chinese Patent Application No. 202311249502.3, filed with the China National Intellectual Property Administration on September 26, 2023, and entitled "BENZONASE NUCLEASE VARIANT WITH IMPROVED SALT TOLERANCE AND USE THEREOF", which is incorporated herein by reference in its entirety.

### TECHNICAL FIELD

The present invention relates to enzyme engineering, in particular to a universal nuclease variant with improved salt tolerance, a preparation method thereof and use thereof.

### BACKGROUND ART

Universal nuclease, also known as non-restrictive endonuclease, mainly functions to degrade nucleic acids in biological products. It can not only reduce the viscosity of cell supernatant and cell lysate to improve efficiency of protein purification and functional research in scientific research, but also be used as a removal reagent for the residual nucleic acid of the host in virus purification, vaccine production, and pharmaceutical industry of proteins and polysaccharides, reducing the residual nucleic acid of the host to pg level so as to improve efficacy and safety of biological products. In addition, it can effectively prevent agglomeration of human peripheral blood mononuclear cells (PBMC) in cell therapy and vaccine research, and is an indispensable tool enzyme in the CGT field.

At present, the most popular universal nuclease is a bacterial nuclease from *Serratia marcescens* disclosed by Benedik and Strych in 1998 (FEMS Microbiol Lett. 165:1-13), which is registered as a trademark Benzonase^{®} by Merck & Co., Inc. Its optimal activity temperature is 37°C, but its major disadvantage is limited tolerance to increased salt concentrations. Data show that sodium ions or potassium ions exert strong inhibition on the activity of this nuclease. When salt concentration in the reaction system is higher than 300 mM, the enzyme activity is almost completely lost. This property makes universal nuclease only functional in salt-free or low-salt environments, which limits its wide application. Since varying concentrations of salt are added to all buffer systems to ensure process stability both in industrial preparation of biological products and in processes for obtaining recombinant proteins in scientific research, there is an urgent market demand currently to develop and produce a universal nuclease with significantly improved salt tolerance.

Enzymes from halophilic microorganisms make it possible to discover salt-tolerant enzymes, and several salt-tolerant nucleases derived from marine microorganisms have been reported so far. Marcin et al. disclosed a novel thermolabile nuclease in 2022 (CN114651062A), which can tolerate 500 mM salt concentration. However, this enzyme maintains high activity only at low temperatures, especially at 4°C to 8°C, and its yield is very low, resulting in high production costs and demanding application environments. In addition, Salt Active nuclease (Yeasen Biotech Co., Ltd., China) is commercially available, which shows optimal activity in 500 mM NaCl, but its relative specific activity only reaches about 25% of the optimal specific activity of Benzonase. Moreover, when salt concentration is lower than 500 mM, its activity decreases obviously with the decrease of salt concentration, indicating a narrow tolerance range for the salt concentration. These characteristics limit its application only at 500 mM salt concentration. Therefore, equivalent enzymes from halophilic organisms are not always available.

In view of the above, the present invention is proposed.

### SUMMARY

In view of the above technical problems, the present invention obtains a universal nuclease variant capable of tolerating a broader salt concentration range through rational design, which solves the pain point of narrow salt tolerance range of universal nucleases currently on the market, enables it to have wider application scenarios and stronger universality, and can achieve nucleic acid removal effect in systems with different salt concentrations.

Specifically, based on the Benzonase universal nuclease, the present invention improves its tolerance range to salt concentrations, especially from 200mM to 500mM, and finally provides a nuclease with broader salt concentration tolerance without compromising its original activity. The present invention not only retains the inherent advantages of Benzonase, such as high activity, stability and high yield in salt-free or very low-salt conditions, but also improves its tolerance to higher salt concentrations.

Therefore, the present invention at least includes the following four objects.

A first object of the present invention is to provide a universal nuclease variant with improved salt tolerance.

A second object of the present invention is to provide the use of the universal nuclease variant with improved salt tolerance in nucleic acid treatment.

A third object of the present invention is to provide a method for preparing the universal nuclease variant with improved salt tolerance.

A fourth object of the present invention is to provide a method of using the universal nuclease variant with improved salt tolerance.

To achieve the above objects, the present invention provides the following technical solutions.

The present invention first provides a universal nuclease with improved salt tolerance, also named as a universal nuclease variant, wherein the variant comprises alteration of at least one or more amino acid sites in a sequence of Benzonase.

Further, the sites comprise any one or more of site 56, site 98, site 101 and/or site 242.

Further preferably, the sites comprise site 98, and further comprise any one or more of site 56, site 101 and/or site 242.

Further, the alteration comprises any one or more of T56D, T98K, N101H and/or G242D.

Further preferably, the alteration comprises T98K, and further comprises any one or more of T56D, N101H and/or G242D.

Further, the Benzonase is a wild-type Benzonase.

Further preferably, the amino acid sequence of the wild-type Benzonase is as set forth in SEQ ID NO. 1.

In some embodiments, the specific sequence of the variant comprises any one or more of SEQ ID NO. 2 to SEQ ID NO. 16.

The present invention also provides a method for improving salt tolerance of a universal nuclease, comprising introducing alteration of at least one or more amino acid sites in a sequence of Benzonase.

Further, the sites comprise any one or more of site 56, site 98, site 101 and/or site 242.

Further preferably, the sites comprise site 98, and further comprise any one or more of site 56, site 101 and/or site 242.

Further, the alteration comprises any one or more of T56D, T98K, N101H and/or G242D.

Further preferably, the alteration comprises T98K, and further comprises any one or more of T56D, N101H and/or G242D.

Further, the Benzonase is a wild-type Benzonase.

Further preferably, the amino acid sequence of the wild-type Benzonase is as set forth in SEQ ID NO. 1.

In some embodiments, the specific sequence of any one or more variants is as set forth in SEQ ID NO. 2 to SEQ ID NO. 16.

The present invention also provides an endonuclease gene, wherein the gene encodes any one of the universal nuclease variants described above.

The present invention also provides an expression cassette, plasmid or vector comprising the endonuclease gene described above.

The present invention also provides a host cell comprising the endonuclease gene described above, or the expression cassette, plasmid or vector described above.

The present invention also provides a method for preparing the universal nuclease variant described above, wherein the universal nuclease variant is obtained by expression using the expression cassette, plasmid, vector, host cell or engineered microorganism described above.

In some embodiments, the method comprises obtaining recombinant expression plasmids comprising the variant described above, transforming each recombinant expression plasmid into, for example, *E. coli,* and selecting positive clones to obtain recombinant expression strains expressing wild-type and mutated universal nucleases. Further, after activation, the strains are inoculated into a fermenter and fermented by auto-induction, and the fermentation supernatant is collected.

The present invention also provides use of the universal nuclease variant described above in nucleic acid digestion or removal.

Further preferably, the use is performed at a salt concentration of 0-500 mM.

The present invention also provides a method for nucleic acid digestion or removal, comprising treating nucleic acid with any one of the universal nuclease variants described above; preferably, the treatment is performed at a salt concentration of 0-500 mM.

The beneficial technical effects of the present invention are as follows.
1) Compared with wild-type nuclease, the mutations or mutation combinations provided in the present invention exhibit significantly improved salt tolerance. Wild-type universal nuclease almost completely loses activity under NaCl concentration higher than 300 mM, while the variants can maintain original enzyme activity without reduction under salt-free or very low-salt conditions, and retain more than 80% activity at 200 mM NaCl. Among them, the following eight variants: T98K, T56D/T98K, T98K/N101H, T98K/G242D, T56D/T98K/G242D, N101H/T98K/G242D, T56D/T98K/N101H, T56D/N101H/T98K/G242D show significantly improved activity at 300 mM, 400 mM and 500 mM NaCl, maintaining more than 70%, 50% and 20% activity respectively. This broadens application of universal nuclease under different salt concentrations, reduces dosage required in high-salt buffer systems, and achieves better nucleic acid removal effect.
2) The mutated universal nuclease prepared in the present invention can solve the pain point of narrow salt tolerance range of universal nucleases currently on the market, with wider application scenarios and suitability for industrial application.

### BRIEF DESCRIPTION OF THE DRAWINGS

FIG. 1. Three-dimensional structure of wild-type universal nuclease and mutation sites.
FIG. 2. SDS-PAGE image during production of recombinant universal nuclease, wherein M represents protein marker, S represents collected fermentation supernatant, SPFF represents elution peak after sample passes through ion exchange column, and SEC represents elution peak after sample passes through molecular sieve.
FIG. 3. HPLC purity of recombinantly produced universal nuclease.
FIG. 4. Comparison of relative specific activities of wild-type and variant universal nucleases under different salt concentrations, wherein WT represents wild-type universal nuclease, and Competitor Y represents Yeasen high-salt tolerant nuclease.

### DETAILED DESCRIPTION OF EMBODIMENTS

The present invention discloses several universal nuclease variants that can tolerate a broader salt concentration range and retain a certain activity at increased salt concentrations as compared with wild-type universal nuclease. Those skilled in the art can refer to the content herein to implement the application. It is particularly noted that all similar substitutions and modifications are obvious to those skilled in the art and are deemed to be included within the scope of the present invention. The preparation methods and applications of the present invention have been described by way of preferred embodiments. It is obvious that those skilled in the art can make changes, or appropriate modifications and combinations to the preparation methods and applications herein without departing from the content, spirit and scope of the present invention to implement and apply the technology of the present invention. Unless otherwise defined, all technical and scientific terms used herein have the same meaning as commonly understood by those skilled in the art to which the present invention belongs.

The following terms or definitions are provided merely to facilitate understanding of the present invention. These definitions shall not be construed as having a scope less than that understood by those skilled in the art.

Unless otherwise defined hereinafter, all technical and scientific terms used in the detailed description of the present invention are intended to have the same meanings as commonly understood by those skilled in the art. Although the following terms are believed to be well understood by those skilled in the art, the following definitions are nevertheless set forth to better explain the present invention.

As used herein, the terms "include", "comprise", "have", "contain" or "involve" are inclusive or open-ended and do not exclude other unrecited elements or method steps. The term "consist of" is considered a preferred embodiment of the term "comprise". Where a group is defined hereinafter as comprising at least a certain number of embodiments, this shall also be understood as disclosing a group preferably consisting of only those embodiments.

The indefinite or definite article used when referring to a singular noun, such as "a", "an" or "the", includes plural forms of the noun.

The terms "about" and "substantially" in the present invention indicate an accuracy interval that can be understood by those skilled in the art and still ensures the technical effect of the feature in question. The term generally indicates a deviation of ±10%, preferably ±5%, from the indicated value.

The terms "or more", "at least", "more than" and the like, e.g., "at least one", are understood to include, but are not limited to, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or 200, 300, 400, 500, 600, 700, 800, 900, 1000, 2000, 3000, 4000, 5000, or more than the recited values. It also includes any larger numbers or fractions therebetween.

Conversely, the term "no more than" includes every value less than the recited value. For example, "not more than 100 nucleotides" includes 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, and 0 nucleotides. It also includes any smaller numbers or fractions therebetween.

The terms "plurality of", "at least two", "two or more", "at least a second" and the like are understood to include, but are not limited to, at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or 200, 300, 400, 500, 600, 700, 800, 900, 1000, 2000, 3000, 4000, 5000, or more. It also includes any larger numbers or fractions therebetween.

The present invention provides a universal nuclease with improved salt tolerance, also referred to as a universal nuclease variant, wherein the variant comprises alteration of at least one or more amino acid sites in a sequence of Benzonase.

It should be understood that the term "variant" used herein is merely an expression form, and any sequence comprising alteration of one or more amino acid sites in the sequence of Benzonase falls within the scope of the "variant sequence" described herein. Therefore, the variant may include mutant forms as well as non-mutant forms. In addition, the specific source form or preparation method of the variant is not limited. For example, in some specific embodiments, the variant is directly obtained by chemical synthesis; in some other specific embodiments, the variant is directly obtained by recombinant expression; in still other specific embodiments, the variant can be obtained by introducing mutant amino acids into the wild-type sequence through genetic engineering means or genetic methods.

In addition, the term "alteration" used herein includes mutant forms but is not limited to mutations. It should be understood that variant sequences obtained in non-mutant forms will also have the same effects and functions in the present invention, and also fall within the scope of reasonable protection of the present invention.

Typically, the Benzonase applicable herein has at least about 65% sequence identity with the nuclease of SEQ ID NO. 1, more particularly at least about 70%, 75%, 80%, 85%, 90%, 95%, or 99% sequence identity. These homologous differences may be caused by different species sources, but it should be understood that, under the condition that the sequence of the core functional domain is consistent, mutations described herein occurring in these basic sequences are all within the protection scope of the present invention.

In some embodiments, the Benzonase described herein refers to a wild-type Benzonase. In some specific embodiments, the amino acid sequence of the wild-type Benzonase is specifically as set forth in SEQ ID NO. 1. It should be understood that the wild-type sequences of bacterial Benzonase s from *Serratia marcescens* obtained from different sources or different habitats may have individual amino acid differences, but they can also be understood as wild-type sequences. Therefore, the amino acid sequence of the Benzonase of the present invention should theoretically not be limited to the sequence of SEQ ID NO. 1. For wild-type sequences having at least about 85% sequence identity, particularly at least about 90%, 95%, or 99% sequence identify, with the nuclease of SEQ ID NO. 1, provided they satisfy the core functional domain sequence consistency, it can be expected that mutations described herein occurring on these basic wild-type sequences are also within the protection scope of the present invention.

It can be understood from the examples of the present invention that the number of mutation or alteration sites of the present invention is not limited. When comprising one verified mutation site of the present invention: such as T56D or T98K; or any two mutation sites: such as T56D and G242D, or T98K and G242D; or even three sites: such as T56D, T98K and G242D, the experimental data of the present invention have shown that these mutations all have excellent salt tolerance and basic enzyme activity characteristics. Therefore, any variant comprising 1, 2, 3, or 4 mutation sites of the present invention falls within the protection scope of the present invention.

Therefore, compared with the corresponding wild-type Benzonase, the Benzonase variant of the present invention comprises at least one alteration. In some embodiments, the at least one alteration occurs at one or more of sites 56, 98, 101, and 242 of the wild-type sequence of Benzonase (especially corresponding to SEQ ID NO. 1).

In some specific embodiments, the wild-type Benzonase comprises at least one mutation, wherein the at least one mutation is at site 56 corresponding to SEQ ID NO. 1, preferably, the mutation is T56D.

In some more specific embodiments, the mutant sequence is as set forth in SEQ ID NO. 2.

SEQ ID NO. 2: (T56D)
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

In some specific embodiments, the wild-type Benzonase comprises at least one mutation, wherein the at least one mutation is at site 98 corresponding to SEQ ID NO. 1, preferably the mutation is T98K.

In some more specific embodiments, the mutant sequence is as set forth in SEQ ID NO. 3.

SEQ ID NO. 3: (T98K)
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

In some specific embodiments, the wild-type Benzonase comprises at least one mutation, wherein the at least one mutation is at site 101 corresponding to SEQ ID NO. 1, preferably the mutation is N101H.

In some more specific embodiments, the mutant sequence is as set forth in SEQ ID NO. 4.

SEQ ID NO. 4: (N101H)
MRFNNKMLALAALLFAAQASADTLESIDNCAVGCPTGGSSNVSIVRHAYTLN
NNSTTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

In some specific embodiments, the wild-type Benzonase comprises at least one mutation, wherein the at least one mutation is at site 242 corresponding to SEQ ID NO. 1; preferably the mutation is G242D.

In some more specific embodiments, the mutant sequence is as set forth in SEQ ID NO. 5.

SEQ ID NO. 5: (G242D)
MRFNNKMLALAALLFAAQASADTLESIDNCAVGCPTGGSSNVSIVRHAYTLN
NNSTTKFANWVAYHITKDTPASGKTRNWKTDPALNPADTLAPADYTGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

In some specific embodiments, the wild-type Benzonase comprises at least two mutations, wherein the at least one mutation is at sites 56 and 98 corresponding to SEQ ID NO. 1; preferably the mutations are T56D and T98K.

In some more specific embodiments, the mutant sequence is as set forth in SEQ ID NO. 6.

SEQ ID NO. 6: (T56D/T98K)
MRFNNKMLALAALLFAAQASADTLESIDNCAVGCPTGGSSNVSIVRHAYTLN
NNSDTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

In some specific embodiments, the wild-type Benzonase comprises at least two mutations, wherein the at least one mutation is at sites 56 and 242 corresponding to SEQ ID NO. 1; preferably the mutations are T56D and G242D.

In some more specific embodiments, the mutant sequence is as set forth in SEQ ID NO. 7.

SEQ ID NO. 7: (T56D/G242D)
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

In some specific embodiments, the wild-type Benzonase comprises at least two mutations, wherein the at least one mutation is at sites 56 and 101 corresponding to SEQ ID NO. 1.

In some more specific embodiments, the mutant sequence is as set forth in SEQ ID NO. 8; preferably the mutations are T56D and N101H.

SEQ ID NO. 8: (T56D/N101H)
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

In some specific embodiments, the wild-type Benzonase comprises at least two mutations, wherein the at least one mutation is at sites 98 and 242 corresponding to SEQ ID NO. 1; preferably the mutations are T98K and G242D.

In some more specific embodiments, the mutant sequence is as set forth in SEQ ID NO. 9.

SEQ ID NO. 9: (T98K/G242D)
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

In some specific embodiments, the wild-type Benzonase comprises at least two mutations, wherein the at least one mutation is at sites 98 and 101 corresponding to SEQ ID NO. 1; preferably the mutations are T98K and N101H.

In some more specific embodiments, the mutant sequence is as set forth in SEQ ID NO. 10.

SEQ ID NO. 10: (T98K/N101H)
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

In some specific embodiments, the wild-type Benzonase comprises at least two mutations, wherein the at least one mutation is at sites 101 and 242 corresponding to SEQ ID NO. 1; preferably the mutations are G242D and N101H.

In some more specific embodiments, the mutant sequence is as set forth in SEQ ID NO. 11.

SEQ ID NO. 11: (G242D/N101H)
MRFNNKMLALAALLFAAQASADTLESIDNCAVGCPTGGSSNVSIVRHAYTLN
NNSTTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

In some specific embodiments, the wild-type Benzonase comprises at least three mutations, wherein the at least one mutation is at sites 56, 98 and 101 corresponding to SEQ ID NO. 1; preferably the mutations are T56D, T98K and N101H.

In some more specific embodiments, the mutant sequence is as set forth in SEQ ID NO. 12.

SEQ ID NO. 12: (T56D/T98K/N101H)
MRFNNKMLALAALLFAAQASADTLESIDNCAVGCPTGGSSNVSIVRHAYTLN
NNSDTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

In some specific embodiments, the wild-type Benzonase comprises at least three mutations, wherein the at least one mutation is at sites 56, 98 and 242 corresponding to SEQ ID NO. 1; preferably the mutations are T56D, T98K and G242D.

In some more specific embodiments, the mutant sequence is as set forth in SEQ ID NO. 13.

SEQ ID NO. 13: (T56D/T98K/G242D)
MRFNNKMLALAALLFAAQASADTLESIDNCAVGCPTGGSSNVSIVRHAYTLN
NNSDTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

In some specific embodiments, the wild-type Benzonase comprises at least three mutations, wherein the at least one mutation is at sites 98, 101 and 242 corresponding to SEQ ID NO. 1; preferably the mutations are T98K, G242D and N101H.

In some more specific embodiments, the mutant sequence is as set forth in SEQ ID NO. 14.

SEQ ID NO. 14: (T98K/G242D/N101H)
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

In some specific embodiments, the wild-type Benzonase comprises at least three mutations, wherein the at least one mutation is at sites 56, 101 and 242 corresponding to SEQ ID NO. 1; preferably the mutations areT56D, G242D and N101H.

In some more specific embodiments, the mutant sequence is as set forth in SEQ ID NO. 15.

SEQ ID NO. 15: (T56D/N101H/G242D)
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

In some specific embodiments, the wild-type Benzonase comprises at least four mutations, wherein the at least one mutation is at sites 56, 98, 101 and 242 corresponding to SEQ ID NO. 1; preferably the mutations are T56D, T98K, G242D and N101H.

In some more specific embodiments, the mutant sequence is as set forth in SEQ ID NO. 16.

SEQ ID NO. 16: (T56D/T98K/G242D/N101H)
MRFNNKMLALAALLFAAQASADTLESIDNCAVGCPTGGSSNVSIVRHAYTLN
NNSDTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN.

As demonstrated by the data in the examples of the present invention, the following eight mutants-T98K, T56D/T98K, T98K/N101H, T98K/G242D, T56D/T98K/G242D, N101H/T98K/G242D, T56D/T98K/N101H, and T56D/N101H/T98K/G242D-show significantly improved activity under 300 mM, 400 mM, and 500 mM NaCl conditions, retaining at least 70%, 50%, and 20% activity, respectively. Therefore, in some preferred embodiments of the present invention, the wild-type Benzonase comprises at least a mutation at site 98, such as T98K, and further comprises other mutations at one or more sites (T56D/G242D/N101H).

It will also be understood that, on the basis of maintaining the basic enzymatic activity of Benzonase, those skilled in the art may further introduce other known effective site mutations on the basis of the mutations disclosed herein to meet practical optimization and improvement requirements. Such mutations, being introduced on the basis of the present invention, are therefore also within the scope of protection of the present invention, and their corresponding effects can be reasonably expected by those skilled in the art based on basic knowledge of enzyme structure.

The method for improving salt tolerance of a universal nuclease provided herein comprises the step of introducing mutations or alterations including at least one or more amino acid sites into the sequence of Benzonase. These mutations or alterations follow the same logic as described above.

For example, in some embodiments, the sites include site 56, site 98, site 101, and/or site 242; in some preferred embodiments, the sites include site 98, and further include site 56, site 101, and/or site 242.

In some specific embodiments, the mutation includes one or more of T56D, T98K, N101H, and/or G242D; in some preferred embodiments, the mutation includes T98K, and further includes one or more of T56D, N101H, and/or G242D.

The endonuclease gene of the present invention comprises any gene sequence encoding any one of the universal nuclease variants described above.

The expression cassette of the present invention can be an expression cassette comprising any one of the endonuclease genes described above.

The plasmid of the present invention can be a plasmid, preferably an expression plasmid, comprising any one of the endonuclease genes described above.

The vector of the present invention can be a vector, preferably an expression vector, comprising any one of the endonuclease genes described above.

The cell of the present invention is a cell, preferably a host cell, capable of expressing a vector comprising any one of the endonuclease genes described above.

The technical solutions of the present invention will be described clearly and completely hereinafter with reference to the examples. It is obvious that the described examples are part of the examples of the present invention, rather than all of them. Based on the examples of the present invention, all other examples obtained by those of ordinary skill in the art without creative efforts shall fall within the scope of protection of the present invention.

### Example

The technical solutions of the present invention will be described clearly and completely hereinafter with reference to the drawings. It is obvious that the described examples are part of the examples of the present invention, rather than all of them. Based on the examples of the present invention, all other examples obtained by those of ordinary skill in the art without creative efforts shall fall within the scope of protection of the present invention.

### Example 1 Design of Mutants

Previous studies of the present invention have found that mutants with significantly improved salt tolerance can be obtained by mutating certain sites on the basis of wild-type non-specific nuclease Benzonase^{®}. In the specific design of mutants, the preference for amino acid selection on the structural outer surface of salt-tolerant enzymes is investigated, and combined with their three-dimensional structure, the following multiple point mutation strategies are selected through corresponding design and screening. Mutations predicted to confer salt-tolerant properties are introduced into wild-type universal nuclease by site-directed mutagenesis.

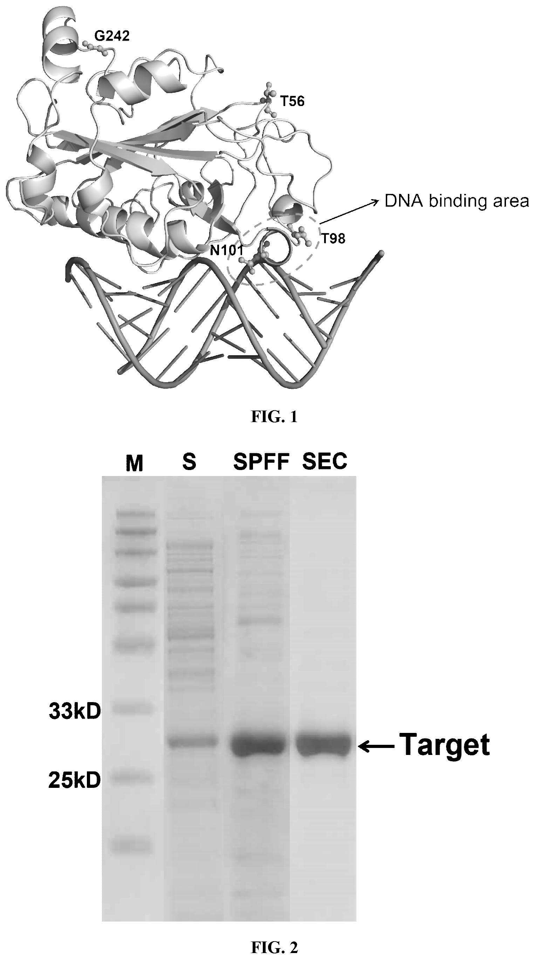

Through batch mutation design and screening, the present invention has finally obtained four optimal mutation sites with a broader potential salt-tolerant range, namely site 56, site 98, site 101 and site 242 (see FIG. 1).

Therefore, on the basis of the wild-type sequence (SEQ ID NO. 1), the present invention designs various combined mutations for verification using sites 56, 98, 101 and 242 as key sites.

SEQ ID NO. 1:

MRFNNKMLALAALLFAAQASADTLESIDNCAVGCPTGGSSNVSIVRHAYTLN
NNSTTKFANWVAYHITKDTPASGKTRNWKTDPALNPADTLAPADYTGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN.

Specifically designed mutation combinations include: T56D/T98K/N101H/G242D, that is, based on SEQ ID NO. 1, designing amino acid sequences that simultaneously possess one or more, or all, of the T56D, T98K, N101H, and G242D mutations.

The detailed information is as follows:

| Group | Mutation Site | Corresponding Sequence |
|---|---|---|
| Group 1 | T56D | SEQ ID NO.2 |
| Group 2 | T98K | SEQ ID NO.3 |
| Group 3 | N101H | SEQ ID NO.4 |
| Group 4 | G242D | SEQ ID NO.5 |
| Group 5 | T56D/T98K | SEQ ID NO.6 |
| Group 6 | T56D/G242D | SEQ ID NO.7 |
| Group 7 | T56D/N101H | SEQ ID NO.8 |
| Group 8 | T98K/G242D | SEQ ID NO.9 |
| Group 9 | T98K/N101H | SEQ ID NO.10 |
| Group 10 | G242D/N101H | SEQ ID NO.11 |
| Group 11 | T56D/T98K/N101H | SEQ ID NO.12 |
| Group 12 | T56D/T98K/G242D | SEQ ID NO.13 |
| Group 13 | T98K/G242D/N101H | SEQ ID NO.14 |
| Group 14 | T56D/N101H/G242D | SEQ ID NO.15 |
| Group 15 | T56D/T98K/G242D/N101H | SEQ ID NO.16 |

The detailed sequence information is as follows:
SEQ ID NO. 2: (T56D) 
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN
SEQ ID NO. 3: (T98K) 
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN
SEQ ID NO. 4: (N101H) 
MRFNNKMLALAALLFAAQASADTLESIDNCAVGCPTGGSSNVSIVRHAYTLN
NNSTTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
 
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN
SEQ ID NO. 5: (G242D) 
MRFNNKMLALAALLFAAQASADTLESIDNCAVGCPTGGSSNVSIVRHAYTLN
NNSTTKFANWVAYHITKDTPASGKTRNWKTDPALNPADTLAPADYTGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN
SEQ ID NO. 6: (T56D/T98K) 
MRFNNKMLALAALLFAAQASADTLESIDNCAVGCPTGGSSNVSIVRHAYTLN
NNSDTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN
SEQ ID NO. 7: (T56D/G242D) 
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN
SEQ ID NO. 8: (T56D/N101H) 
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN
SEQ ID NO. 9: (T98K/G242D) 
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN
SEQ ID NO. 10: (T98K/N101H) 
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN
SEQ ID NO. 11: (G242D/N101H) 
MRFNNKMLALAALLFAAQASADTLESIDNCAVGCPTGGSSNVSIVRHAYTLN
NNSTTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN
SEQ ID NO. 12: (T56D/T98K/N101H) 
MRFNNKMLALAALLFAAQASADTLESIDNCAVGCPTGGSSNVSIVRHAYTLN
NNSDTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWAGLPDDVQASLKSKPGVLPELMGCKN
SEQ ID NO. 13: (T56D/T98K/G242D) 
MRFNNKMLALAALLFAAQASADTLESIDNCAVGCPTGGSSNVSIVRHAYTLN
NNSDTKFANWVAYHITKDTPASGKTRNWKTDPALNPADTLAPADYKGANAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN
SEQ ID NO. 14: (T98K/G242D/N101H) 
MRFNNKMLALAALLFAAQASADTLESIDNCAVGCPTGGSSNVSIVRHAYTLN
NNSTTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN
SEQ ID NO. 15: (T56D/N101H/G242D) 
MRFNNKMLALAALLFAAQASADTLESIDNCAVGCPTGGSSNVSIVRHAYTLN
NNSDTKFANWVAYHITKDTPASGKTRNWKTDPALNPADTLAPADYTGAHAALKVD
RGHQAPLASLAGVSDWESLNYLSNITPQKSDLNQGAWARLEDQERKLIDRADISSVY
 
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN
SEQ ID NO. 16: (T56D/T98K/G242D/N101H)

MRFNNKMLALAALLFAAQASADTLESIDNCAVGCPTGGSSNVSIVRHAYTLN
NNSDTKFANWVAYHITKDTPASGKTRNWKTDPALNPADTLAPADYKGAHAALKVD
RGHQAPLASLAGVSDWESLNYLSNITPQKSDLNQGAWARLEDQERKLIDRADISSVY
TVTGPLYERDMGKLPGTQKAHTIPSAYWKVIFINNSPAVNHYAAFLFDQNTPKGADF
CQFRVTVDEIEKRTGLIIWADLPDDVQASLKSKPGVLPELMGCKN

### Example 2 Production of Recombinant Mutants

Gene fragments corresponding to SEQ ID NO. 1 and SEQ ID NO. 2-16 in Example 1 were respectively inserted into vector pET41a to obtain recombinant expression plasmids. Each recombinant expression plasmid was transformed into competent cells of *E. coli* BL21(DE3) by heat shock. Positive clones were selected and cultured overnight at 37 °C in LB medium containing kanamycin to obtain recombinant expression strains producing wild-type and mutant universal nucleases. 600 µL of the primary recombinant expression bacterial culture was inoculated into 200 mL of LB medium containing kanamycin and cultured at 37 °C until OD600 reached a range of 1.5-3.5. Animal-free *E. coli* auto-induction medium for large-scale culture was prepared in advance and sterilized. The fermented secondary seed culture obtained above was inoculated into the prepared medium at a volume ratio of 1.4%. The fermentation was set at 37 °C and a rotation speed of 200 rpm; pressure and air flow rate were adjusted as appropriate. The pH was monitored during fermentation, and fermentation was terminated after 11 h of culture. The supernatant of the fermentation broth was collected by centrifuge at 4000 rpm for 15 min.

The obtained supernatant was then adjusted to pH 6.0 and filtered using a 0.22 µm filter membrane. Concentration and diafiltration were then performed: after concentrating by 4-5 folds, the sample was diluted with equilibration buffer (20 mM NaAC, 2 mM MgCl₂, 20% glycerol, pH 6.0), reconcentrated to the original volume, and diluted and concentrated again, and this cycle was repeated 4-5 times. The sample after concentration and diafiltration was loaded onto a cation exchange column. The target protein bound to the column was eluted with buffer containing 20 mM NaAC, 2 mM MgCl₂, 350 mM NaCl, and 20% glycerol (pH 6.0). To obtain protein with a single conformation, the peak of the previous elution and collection was further subjected to a molecular sieve and eluted with buffer containing 25 mM Tris, 5 mM MgCl₂, 500 mM NaCl, and 20% glycerol (pH 7.5), and collected in fractions and subjected to SDS-PAGE and HPLC analysis.

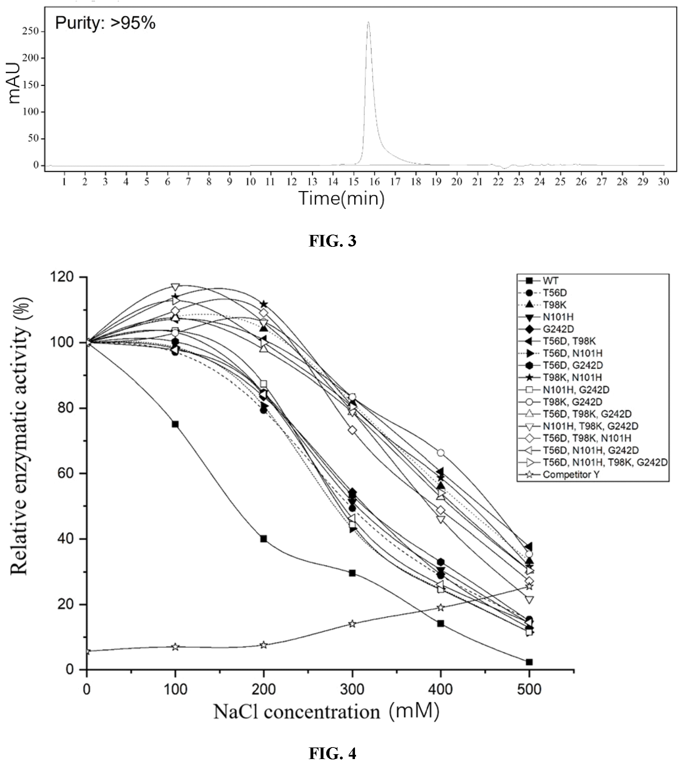

According to the detection results (exemplified in FIG. 2 and FIG. 3), peaks with purity higher than 95% by both SDS-PAGE and HPLC were combined, supplemented with glycerol to 50%, and stored at -20 °C for later use. Protein concentration was determined by spectrophotometry at a wavelength of 280 nm.

### Example 3 Comparison of Enzymatic Activity of Wild-Type and Mutants at Different Salt Concentrations

To determine the improvement in activity of the mutated universal nuclease at different salt concentrations, the wild-type and mutants were each diluted to an appropriate fold, such that the detected values after dilution fall within the linear range. First, 400 µL of salmon sperm DNA (1 mg/mL) was taken as substrate, then an equal volume of diluted enzyme solution was added, and finally the reaction system was adjusted to 500 µL with reaction buffer (50 mM Tris, 5 mM MgCl₂, 0.1 mg/mL BSA, pH 8.0). After evenly mixing, the reaction solution was incubated in a 37 °C water bath for 30 min. After incubation, 500 µL of 20% trichloroacetic acid was rapidly added in sequence in the reaction solution to terminate the reaction. After an ice-water bath for at least 10 min, 200 µL of each reaction solution was added into a centrifuge tube containing 1800 µL of double-distilled water and mixed evenly. The absorbance of the diluted solution at 260 nm was detected with a UV spectrophotometer. One unit (U) of activity was defined as the amount of enzyme required to increase the absorbance at 260 nm by 1.0 at 37 °C for 30 min in the presence of excess substrate. By adjusting the NaCl concentration in the reaction system to 0 mM, 100 mM, 200 mM, 300 mM, 400 mM, and 500 mM, respectively, the enzymatic activity of the universal nuclease at different salt concentrations was determined according to the above embodiment, and the specific activity was calculated based on the detection results.

The results are shown in FIG. 4: the wild-type universal nuclease is sensitive to salt, retaining only 40% and 30% relative enzymatic activity at 200 mM and 300 mM NaCl, respectively, and almost losing activity at NaCl concentrations higher than 300 mM. Compared with the wild-type, the mutants can maintain the original enzymatic activity without reduction under salt-free or very low-salt conditions, and meanwhile show significantly improved salt tolerance.

Moreover, it can be seen from FIG. 4 that all mutants can maintain 80% or even higher activity at 200 mM NaCl, which is significantly improved compared with the wild-type. Among them, the following eight mutants: T98K, T56D/T98K, T98K/N101H, T98K/G242D, T56D/T98K/G242D, N101H/T98K/G242D, T56D/T98K/N101H, T56D/N101H/T98K/G242D show significantly improved activity at 300 mM, 400 mM and 500 mM NaCl, retaining at least 70%, 50% and 20% or even higher activity, respectively. All the above mutants contain the T98K mutation, indicating the importance of this mutation for improving the salt tolerance of the universal nuclease. The other seven mutants: T56D, N101H, G242D, T56D/N101H, T56D/G242D, N101H/G242D, T56D/N101H/G242D show limited improvement in activity at 300 mM, 400 mM and 500 mM NaCl, retaining at least 43%, 25%, 11% or even higher activity, respectively, which is still improved compared with the wild-type.

Compared with commercial products in the prior art, such as Salt Active nuclease (Yeasen Biotechnology, China), in which it exhibits optimal enzymatic activity at 500 mM, but the relative enzymatic activity only reaches about 25% of the optimal enzymatic activity of the wild-type universal nuclease (0 mM NaCl), and the relative enzymatic activity is much lower than 25% at NaCl concentrations lower than 500 mM, the variants screened in the present invention not only have relative enzymatic activity much higher than 25% at NaCl concentrations lower than 500 mM, but also even some variants have better enzymatic activity than the commercial Salt Active nuclease at 500 mM NaCl.

In summary, the novel universal nuclease variants screened in the present invention have a wider application range and better activity.

The results of the above examples show that mutating amino acids at specific sites on the basis of the wild-type sequence can effectively increase the tolerance of universal nuclease at different salt concentrations, so that it can be more widely used in environments containing different salt concentrations. The mutant with the most mutation sites achieves good effects compared with the wild-type, but at the same time, some single, double and triple mutations can also achieve similar effects. This indicates that the number of mutations is not necessarily the more the better, and the key is to find out the amino acid sites that play a decisive role.

The foregoing description of the specific exemplary embodiments of the present invention is for the purpose of illustration and exemplification. These descriptions are not intended to limit the present invention to the precise forms disclosed, and obviously many changes and modifications can be made in light of the above teaching. The exemplary embodiments were chosen and described in order to explain the specific principles of the present invention and their practical application, so as to enable those skilled in the art to implement and utilize various different exemplary embodiments and various different selections and modifications of the present invention. The scope of the present invention is intended to be defined by the claims and their equivalents.

## Claims

1. A universal nuclease variant with improved salt tolerance, **characterized in that** the variant comprises alteration of at least one or more amino acid sites in a sequence of Benzonase.

2. A method for improving salt tolerance activity of a universal nuclease, **characterized by** comprising introducing alteration of at least one or more amino acid sites in a sequence of Benzonase.

3. The universal nuclease variant according to claim 1 or the method according to claim 2, wherein the sites comprise any one or more of site 56, site 98, site 101 and/or site 242;
preferably, the sites comprise site 98, and further comprise any one or more of site 56, site 101 and/or site 242.

4. The universal nuclease variant according to claim 1 or the method according to claim 2, wherein the alteration comprises any one or more of T56D, T98K, N101H and/or G242D;
preferably, the alteration comprises T98K, and further comprises any one or more of T56D, N101H and/or G242D.

5. The universal nuclease variant according to claim 1 or the method according to claim 2, wherein the Benzonase is a wild-type Benzonase;
preferably, a sequence of the wild-type Benzonase is as set forth in SEQ ID NO. 1.

6. An endonuclease gene, **characterized in that** the gene encodes the universal nuclease variant according to any one of claims 1, 3-5.

7. An expression cassette, plasmid, vector, host cell or engineered microorganism, **characterized by** comprising the endonuclease gene according to claim 6.

8. A method for preparing a universal nuclease variant, **characterized in that** the universal nuclease variant is obtained by expression using the expression cassette, plasmid, vector, host cell or engineered microorganism according to claim 7.

9. Use of the universal nuclease variant according to any one of claims 1, 3-5 in nucleic acid digestion or removal.

10. A method for nucleic acid digestion or removal, **characterized by** comprising treating nucleic acid with the universal nuclease variant according to any one of claims 1, 3-5; preferably, the treatment is performed at a salt concentration of 0-500 mM.
